# EUROPÄISCHE PATENTSCHRIFT

(11) **EP 2 981 463 B1**
(45) Veröffentlichungstag und Bekanntmachung des Hinweises auf die Patenterteilung: **01.06.2022**
(21) Anmeldenummer: 14715262.3
(22) Anmeldetag: 01.04.2014
(51) Int. Cl.: B64D 11/00, B64D 11/06

(54) **SITZVORRICHTUNG**
SEATING APPARATUS
DISPOSITIF À SIÈGE

(30) Priorität: 05.04.2013 DE 102013103436
(43) Veröffentlichungstag der Anmeldung: 10.02.2016
(73) Patentinhaber: Recaro Aircraft Seating GmbH & Co. KG, 74523 Schwäbisch Hall (DE)
(72) Erfinder: GUTTROPF, Roland, 74572 Blaufelden (DE); IRIMIA, Cristian, 74523 Schwäbisch Hall (DE); KRAUTH, Hubert, 74535 Mainhardt (DE)
(74) Vertreter: Daub, Thomas
(86) Internationale Anmeldenummer: PCT/EP2014/056526
(87) Internationale Veröffentlichungsnummer: WO 2014/161853

(56) Entgegenhaltungen:
- EP-A1- 2 465 722
- EP-A2- 1 600 376
- US-A1- 2002 175 554
- US-A1- 2012 091 764
- US-A1- 2013 038 103

## Beschreibung

### Stand der Technik

Die Erfindung betrifft eine Sitzvorrichtung nach dem Oberbegriff des Patentanspruchs 1.

Aus der Druckschrift US 2002/0175554 A1 ist bereits eine Sitzvorrichtung, insbesondere Flugzeugsitzvorrichtung, mit zumindest einer seitlichen Gepäckstange, die dazu vorgesehen ist, unterhalb eines Sitzes an einer, einem Gang einer Passagierkabine zugewandten Seite des Sitzes angebracht zu werden, wobei sich die seitliche Gepäckstange (10a; 10b) von ihrem vorderen Ende aus im Wesentlichen parallel zu dem Kabinenboden in Richtung eines hinteren Bereichs des Sitzes erstreckt, und mit zumindest einem Trägerelement (14a; 14b), das dazu vorgesehen ist, eine Abstützkraft aufzunehmen, bekannt.

Die Aufgabe der Erfindung besteht insbesondere darin, eine gattungsgemäße Vorrichtung mit verbesserten Eigenschaften hinsichtlich Komplexität und Gewicht bereitzustellen. Die Aufgabe wird erfindungsgemäß durch die Merkmale des Patentanspruchs 1 gelöst, während vorteilhafte Ausgestaltungen und Weiterbildungen der Erfindung den Unteransprüchen entnommen werden können.

### Vorteile der Erfindung

Die Erfindung geht aus von einer Sitzvorrichtung, insbesondere einer Flugzeugsitzvorrichtung, mit zumindest einer seitlichen Gepäckstange, die dazu vorgesehen ist, unterhalb eines Sitzes an einer, einem Gang einer Passagierkabine zugewandten Seite des Sitzes angebracht zu werden, wobei sich die seitlichen Gepäckstange von ihrem vorderen Ende aus im Wesentlichen parallel zu dem Kabinenboden in Richtung eines hinteren Bereichs des Sitzes erstreckt, und mit zumindest einem Trägerelement, das dazu vorgesehen ist, in zumindest einem Betriebszustand eine Abstützkraft aufzunehmen.

Es wird vorgeschlagen, dass das Trägerelement von einem Trittelement gebildet ist, das einen von einem Kabinenboden erhöhten Trittbereich bereitstellt, und dass das Trägerelement und die Gepäckstange zumindest eine Koppelstelle aufweisen, über die die Gepäckstange und das Trägerelement form-, stoff- und/oder kraftschlüssig miteinander verbunden sind und die dazu vorgesehen ist, zumindest einen Teil der Abstützkraft von dem Trägerelement auf die Gepäckstange zu übertragen.

Unter einer "Gepäckstange" soll dabei insbesondere ein Element verstanden werden, das unterhalb eines Sitzes, insbesondere unterhalb eines Flugzeugsitzes, angeordnet und dazu vorgesehen ist, zu verhindern, dass lose Elemente, wie insbesondere Gepäck, unter dem Sitz hindurchgelangen können und so beispielsweise von einer Sitzreihe in die nächste gelangen können. Dabei erstreckt sich die Gepäckstange von einer Unterseite des entsprechenden Sitzes in Richtung einer Aufständerebene, auf der der Sitz aufgeständert ist, im Falle des Flugzeugsitzes also in Richtung eines Kabinenbodens.

Unter einer "seitlichen Gepäckstange" soll dabei insbesondere ein Teil einer Gepäckstange, die an einer, einem Gang einer Passagierkabine zugewandten Seite des Sitzes angeordnet ist, verstanden werden. Dabei kann sich die gesamte Gepäckstange auch in andere Bereiche unterhalb des Sitzes erstrecken und beispielsweise in einer Querrichtung des Sitzes zu einer gegenüberliegenden Seite des Sitzes verlaufen. Unter "unterhalb eines Sitzes" soll dabei insbesondere von einem Sitzboden des Sitzes in Richtung einer Aufständerebene, auf der der Sitz aufgeständert ist, gesehen verstanden werden. Unter "vorgesehen" soll insbesondere speziell ausgelegt und/oder ausgestattet verstanden werden. Darunter, dass ein Objekt zu einer bestimmten Funktion vorgesehen ist, soll insbesondere verstanden werden, dass das Objekt diese bestimmte Funktion in zumindest einem Anwendungs- und/oder Betriebszustand erfüllt und/oder ausführt. Unter einem "Trägerelement" soll in diesem Zusammenhang insbesondere ein Funktionselement verstanden werden, das für eine bestimmte Funktion und/oder zur Anbindung von zumindest einem Element, wie beispielsweise einem Lichtelement, einem Halteelement, einem Betätigungselement und/oder einem anderen, dem Fachmann als sinnvoll erscheinenden Element, vorgesehen ist. Unter "eine Abstützkraft aufnehmen" soll dabei insbesondere verstanden werden, dass das Trägerelement eine durch eine Funktion des Trägerelements auftretende Kraft, wie beispielsweise eine Gewichtskraft, aufnimmt und zerstörungsfrei ableitet. Unter einer "Koppelstelle" soll dabei insbesondere eine Stelle verstanden werden, an der zwei Elemente, insbesondere die Gepäckstange und das Trägerelement, in zumindest eine Richtung, vorzugsweise in zumindest zwei Richtungen starr miteinander verbunden sind. Dabei sind die zwei über die Koppelstelle miteinander verbundenen Elemente zumindest in eine Richtung translatorisch und vorzugsweise zusätzlich um eine Achse rotatorisch miteinander gekoppelt. Dabei können die Elemente über die Koppelstelle form-, stoff- und/oder kraftschlüssig miteinander verbunden sein. Dadurch kann das Trägerelement besonders einfach an einen Sitz, insbesondere an einen Flugzeugsitz, angebracht werden, wobei insbesondere ein vorteilhaft geringes Gewicht der Sitzvorrichtung erreicht werden kann und das Trägerelement mittels einer besonders vorteilhaft geringen Anzahl von Bauteilen an dem Sitz befestigt werden kann.

Unter einem "Trittelement" soll insbesondere ein Element verstanden werden, das einen Trittbereich für eine Person bereitstellt, die insbesondere einen von einem Untergrund, insbesondere von dem Kabinenboden, erhöhten Tritt bereitstellt, über den eine Person einen höher gelegenen Bereich, wie insbesondere über dem entsprechenden Sitz angeordnete Verstaufächer, einfach und bequem erreichen kann. Dadurch kann das Trägerelement besonders vorteilhaft ausgebildet werden und eine Trittmöglichkeit, wie insbesondere der so genannte Stewardstep, kann besonders vorteilhaft in den Sitz integriert und an ihm befestigt werden.

Ferner wird vorgeschlagen, dass das Trägerelement eine im Wesentlichen L-förmige Grundform aufweist. Dadurch kann das Trägerelement insbesondere zur Anbringung an den Sitz besonders vorteilhaft ausgebildet werden.

Unter "form- und/oder kraftschlüssig verbunden" soll dabei insbesondere eine lösbare Verbindung verstanden werden, wobei eine Haltekraft zwischen zwei Bauteilen vorzugsweise durch einen geometrischen Eingriff der Bauteile ineinander und/oder eine Reibkraft zwischen den Bauteilen übertragen wird. Unter "formschlüssig" soll insbesondere verstanden werden, dass aneinander liegende Flächen von miteinander formschlüssig verbundenen Bauteilen eine in Normalenrichtung der Flächen wirkende Haltekraft aufeinander ausüben. Insbesondere befinden sich die Bauteile in einem geometrischen Eingriff miteinander. Dadurch kann das Trägerelement besonders einfach und mechanisch zerstörungsfrei trennbar mit der Gepäckstange verbunden werden.

Weiterhin wird vorgeschlagen, dass das Trägerelement ein Formschlusselement umfasst, das die Gepäckstange zur formschlüssigen Verbindung zumindest im Wesentlichen umschließt. Unter einem "Formschlusselement" soll dabei insbesondere ein Element verstanden werden, das mittels eines geometrischen Eingriffs mit einem korrespondierenden Formschlusselement und/oder einer Auflage auf einem korrespondierenden Formschlusselement eine formschlüssige Verbindung eingehen kann. Unter "zumindest im Wesentlichen umschließt" soll dabei insbesondere verstanden werden, dass das Formschlusselement die Gepäckstange zumindest zu 50%, vorzugsweise zu mehr als 75% und in einem besonders vorteilhaften Zustand in einer Richtung vollständig umschließt. Dadurch kann das Trägerelement besonders einfach und vorteilhaft mit der Gepäckstange verbunden zu werden.

Zudem wird vorgeschlagen, dass die Gepäckstange zumindest ein Formschlusselement umfasst, das korrespondierend zu dem Formschlusselement des Trägerelements ausgebildet ist. Unter "korrespondierend ausgebildet sein" soll insbesondere verstanden werden, dass das Formschlusselement der Gepäckstange entsprechend dem anderen korrespondierenden Formschlusselement ausgebildet ist, sodass die beiden Formschlusselemente formschlüssig miteinander verbindbar sind. Dadurch kann das Trägerelement besonders einfach mittels der Formschlusselemente mit der Gepäckstange verbunden werden.

Weiter wird vorgeschlagen, dass das zumindest eine Formschlusselement der Gepäckstange zumindest eine an die Gepäckstange angeformte Erhebung aufweist. Unter einer "angeformten Erhebung" soll insbesondere eine Erhebung verstanden werden, die stoffschlüssig mit der Gepäckstange verbunden und dabei vorzugsweise einstückig mit der Gepäckstange ausgebildet ist. Grundsätzlich ist es auch denkbar, dass das Formschlusselement der Gepäckstange, das korrespondierend zu dem Formschlusselement des Trägerelements ausgebildet ist, als ein von der Gepäckstange separat ausgebildetes Element ausgebildet ist, wie beispielsweise als ein Bolzen, der in eine Aufnahme der Gepäckstange eingeführt werden kann. Dadurch kann das Formschlusselement der Gepäckstange besonders einfach ausgebildet werden.

Ferner wird vorgeschlagen, dass die Gepäckstange zumindest teilweise von einem Vollprofil gebildet ist. Unter zumindest teilweise von einem Vollprofil gebildet" soll dabei insbesondere verstanden werden, dass die Gepäckstange zumindest zu 50%, vorzugsweise zu 80% und in einer besonders vorteilhaften Ausgestaltung zu 100% von einem Vollprofil gebildet ist. Dadurch kann die Gepäckstange besonders vorteilhaft ausgebildet werden.

Weiterhin wird vorgeschlagen, dass die Gepäckstange zumindest teilweise als eine flache Stange ausgebildet ist. Unter einer "flachen Stange" soll insbesondere eine Stange verstanden werden, die von einem Vollprofil gebildet ist und dabei einen rechteckigen Querschnitt aufweist, wobei eine Höhe des Querschnitts dabei wesentlich, vorzugsweise zumindest um das Zweifache größer ist als eine Breite. Dadurch kann die Gepäckstange von einem besonders vorteilhaften Vollprofil gebildet werden, das insbesondere platzsparend ausgebildet ist.

Es wird weiter vorgeschlagen, dass die Gepäckstange und das Trägerelement einstückig miteinander ausgebildet sind. Unter "einstückig" soll insbesondere zumindest stoffschlüssig verbunden verstanden werden, beispielsweise durch einen Schweißprozess, einen Klebeprozess, einen Anspritzprozess und/oder einen anderen, dem Fachmann als sinnvoll erscheinenden Prozess, und/oder vorteilhaft in einem Stück geformt verstanden werden, wie beispielsweise durch eine Herstellung aus einem Guss und/oder durch eine Herstellung in einem Ein- oder Mehrkomponentenspritzverfahren und vorteilhaft aus einem einzelnen Rohling oder einer Rohmasse. Dadurch können die Gepäckstange und das Trägerelement besonders vorteilhaft gemeinsam ausgebildet werden, wodurch insbesondere vorteilhaft auf eine Verbindung des Trägerelements mit der Gepäckstange über Formschlusselemente verzichtet werden kann. Dabei sind das Trägerelement und die Gepäckstange aus einem Metall einstückig miteinander ausgebildet, vorzugsweise aus einem Leichtmetall, wie insbesondere einem Aluminium. Dabei ist es ebenso denkbar, dass das Trägerelement und die Gepäckstange, die einstückig miteinander ausgebildet sind, aus einem anderen, dem Fachmann als sinnvoll erscheinenden Material gebildet sind, wie beispielsweise einem Kunststoff, wie insbesondere vorteilhaft aus einem Faserverbundkunststoff. Dabei ist es ebenfalls denkbar, dass das Trägerelement und die Gepäckstange aus mehreren Komponenten aufgebaut sind und aus mehreren miteinander verbundenen, unterschiedlichen Materialien bestehen.

Die erfindungsgemäße Sitzvorrichtung soll hierbei nicht auf die oben beschriebene Anwendung und Ausführungsform beschränkt sein. Insbesondere kann die erfindungsgemäße Sitzvorrichtung zu einer Erfüllung einer hierin beschriebenen Funktionsweise eine von einer hierin genannten Anzahl von einzelnen Elementen, Bauteilen und Einheiten abweichende Anzahl aufweisen.

Es zeigen:
- Fig. 1: eine Seitenansicht eines Teils eines Sitzes mit einer erfindungsgemäßen Sitzvorrichtung in einem ersten Ausführungsbeispiel,
- Fig. 2: eine schematische Ansicht der erfindungsgemäßen Sitzvorrichtung,
- Fig. 3: die Sitzvorrichtung mit einer Gepäckstange und einem Trittelement während einer Montage,
- Fig. 4: eine Detailansicht eines Formschlusselements der Gepäckstange,
- Fig. 5: eine Detailansicht einer Anbindung der Gepäckstange an eine vordere Querstange des Sitzes und
- Fig. 6: eine schematische Darstellung einer erfindungsgemäßen Sitzvorrichtung in einem zweiten Ausführungsbeispiel.

### Beschreibung der Ausführungsbeispiele

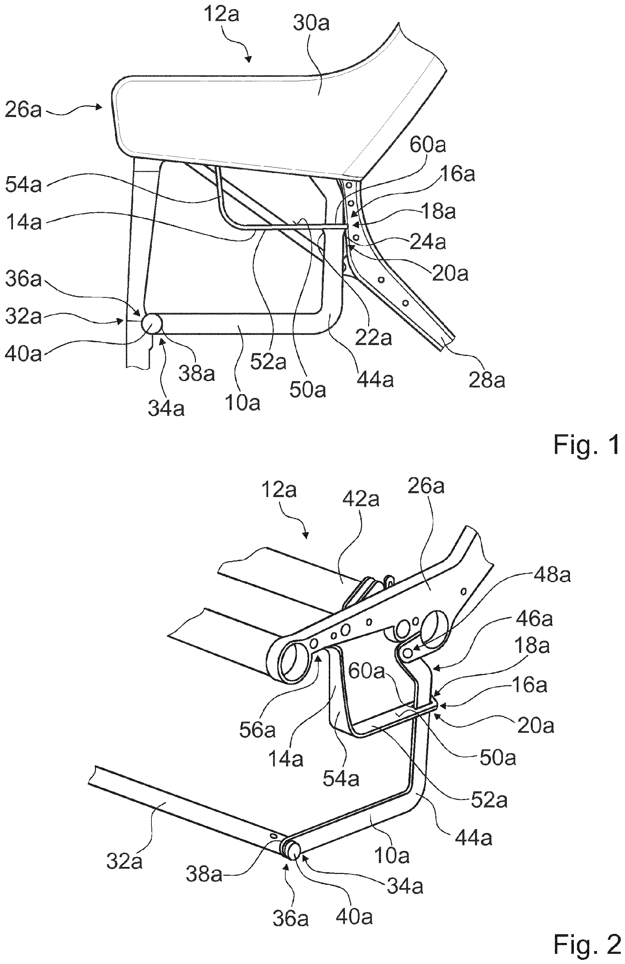

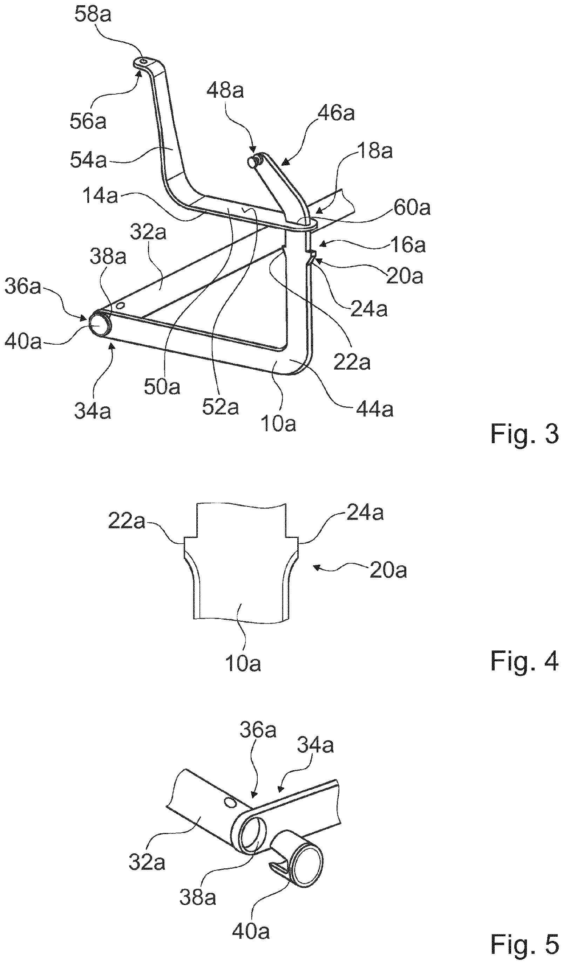

Die Figuren 1 bis 5 zeigen ein erstes Ausführungsbeispiel einer erfindungsgemäßen Sitzvorrichtung. Die Sitzvorrichtung ist Teil eines nur teilweise dargestellten Sitzes 12a. Der Sitz 12a ist als ein Flugzeugsitz ausgebildet. Die Sitzvorrichtung ist als eine Flugzeugsitzvorrichtung ausgebildet. Der als Flugzeugsitz ausgebildete Sitz 12a weist eine nicht näher dargestellte Rückenlehne zu einer Abstützung eines Rückens eines Gasts und einen nicht näher dargestellten Sitzboden, der eine Sitzfläche für den Gast ausbildet, auf. Der Sitz 12a weist eine Aufständereinheit 28a auf, an der die Rückenlehne und der Sitzboden angeordnet sind. Zudem ist der als Flugzeugsitz ausgebildete Sitz 12a mittels der Aufständereinheit 28a auf einem Kabinenboden einer Passagierkabine aufgeständert. Außerdem weist der Sitz 12a seitlich der Sitzflächen jeweils einen Sitzteiler 26a auf, der die einzelnen Sitze 12a jeweils seitlich begrenzt und Sitze 12a einer Sitzreihe voneinander trennt. Der Sitzteiler 26a ist dabei zur Anbringung einer Armlehne für die entsprechenden Sitze, die der Sitzteiler 26a seitlich begrenzt, vorgesehen. In montiertem Zustand ist der Sitzteiler 26a von einer Abdeckung 30a abgedeckt.

Die Sitzvorrichtung umfasst eine seitliche Gepäckstange 10a. Die seitliche Gepäckstange 10a ist Teil des als Flugzeugsitz ausgebildeten Sitzes 12a, der mit einer Seite an einen Gang in der Passagierkabine grenzt. Der als Flugzeugsitz ausgebildete Sitz 12a weist dabei weitere, nicht näher dargestellte Gepäckstangen auf, die an anderen Stellen des Sitzes 12a angeordnet sind. Dabei ist es denkbar, dass die Gepäckstangen gleich ausgebildet sind wie die im Folgenden beschriebene Gepäckstange 10a, es ist aber grundsätzlich auch denkbar, dass die anderen, nicht näher dargestellten Gepäckstangen unterschiedlich zu der dargestellten seitlichen Gepäckstange 10a ausgebildet sind. Die Gepäckstange 10a ist unterhalb des Sitzes 12a angebracht. Die Gepäckstange 10a ist in einem Bereich zwischen dem Sitzboden des Sitzes 12a und dem Kabinenboden, auf dem der als Flugzeugsitz ausgebildete Sitz 12a aufgeständert ist, angeordnet und verhindert dadurch, dass Elemente, wie insbesondere Gepäckstücke, die in einem Fußraum des Gastes gelagert sind, unterhalb des Sitzes 12a durchrutschen können. Die Gepäckstange 10a weist dabei eine L-förmige Erstreckung auf. Der Sitz 12a weist eine vordere Querstange 32a auf, die einen Grundrahmen des Sitzes 12a ausbildet und die in montiertem Zustand in der Nähe des Kabinenbodens angeordnet ist. Die vordere Querstange 32a ist ebenfalls als eine Gepäckstange ausgebildet. Die vordere Querstange 32a weist in montiertem Zustand einen Abstand zu dem Kabinenboden auf, der geringer ist als ein Abstand zu dem Sitzboden des Sitzes 12a. Die Gepäckstange 10a weist ein vorderes Ende 34a auf, das über eine Verbindungsstelle 36a fest mit der vorderen Querstange 32a verbunden ist. Grundsätzlich ist es dabei auch denkbar, dass die Gepäckstange 10a und die Querstange 32a einstückig miteinander ausgebildet sind und so eine gemeinsame, große Gepäckstange ausbilden. Die Gepäckstange 10a weist ein Formschlusselement 38a auf, das als Wandung eines Durchgangslochs ausgebildet ist, mittels dessen die Gepäckstange 10a fest mit der vorderen Querstange 32a verbunden ist. Zur Verbindung des vorderen Endes 34a der Gepäckstange 10a mit der vorderen Querstange 32a weist die Sitzvorrichtung ein separat ausgebildetes Formschlusselement 40a auf, das über das Formschlusselement 38a der Gepäckstange 10a fest mit der Gepäckstange 10a gekoppelt wird und seitlich in die als Hohlprofil ausgebildete vordere Querstange 32a formschlüssig eingebracht wird. Dadurch ist die Gepäckstange 10a über das Formschlusselement 40a fest mit der vorderen Querstange 32a verbunden. Grundsätzlich ist es dabei auch denkbar, dass das Formschlusselement 40a einstückig an die Gepäckstange 10a oder die Querstange 32a angeformt ist. Dabei würden die Gepäckstange 10a und die Querstange 32a über das einstückig angeformte Formschlusselement 40a direkt miteinander verbunden werden. Von dem vorderen Ende 34a aus erstreckt sich die Gepäckstange 10a im Wesentlichen parallel zu dem Kabinenboden in Richtung eines hinteren Bereichs des Sitzes 12a. In einem Bereich eines hinteren Tragrohrs 42a des Sitzes 12a weist die Gepäckstange 10a eine 90 Grad-Umlenkung 44a auf und verläuft von dort an in Richtung des Sitzbodens. In einem Bereich eines hinteren Endes 46a weist die Gepäckstange 10a einen Knick in Richtung der vorderen Querstange 32a auf. Der Knick bildet dabei einen Winkel von kleiner 45 Grad aus. An dem hinteren Ende 46a der Gepäckstange 10a weist die Gepäckstange 10a ein Durchgangsloch auf. Über das Durchgangsloch ist die Gepäckstange 10a an dem seitlichen Sitzteiler 26a des Sitzes 12a anbindbar. Dazu weist der entsprechende Sitzteiler 26a eine Aufnahme auf, die ebenfalls zumindest ein Durchgangsloch aufweist. Über das Durchgangsloch der Aufnahme des Sitzteilers 26a und das Durchgangsloch des hinteren Endes 46a der Gepäckstange 10a ist das hintere Ende 46a der Gepäckstange 10a mittels einer Schraubenverbindung 48a fest mit dem Sitzteiler 26a verbunden. Die Gepäckstange 10a ist dabei von einem Vollprofil gebildet. Die Gepäckstange 10a ist als eine flache Stange ausgebildet, wobei sie einen rechteckigen Querschnitt aufweist. Dabei ist eine Breite des Querschnitts der Gepäckstange 10a in montiertem Zustand parallel zu der vorderen Querstange 32a und damit parallel zu einer Querrichtung des Sitzes 12a ausgerichtet. Die Breite des Querschnitts ist dabei wesentlich kleiner als eine Höhe des Querschnitts, die in montiertem Zustand parallel zu einer Vertikalrichtung des Sitzes 12a ausgebildet ist, die insbesondere orthogonal zu der Querrichtung und dem Kabinenboden ausgerichtet ist. Dabei ist die Gepäckstange 10a beispielsweise in einem Fräsprozess oder in einem Gießverfahren hergestellt. Grundsätzlich ist es auch denkbar, dass die Gepäckstange 10a einen anderen Querschnitt und oder einen anderen Verlauf aufweist. Es ist weiterhin denkbar, dass die als flache Stange ausgebildete Gepäckstange 10a auf einer Seite eine oder mehrere Taschen aufweist, die beispielsweise in die Gepäckstange 10a eingefräst sein können. Die in die Gepäckstange 10a eingebrachten Taschen könnten dabei zu einer Gewichtsreduktion der Gepäckstange 10a beitragen. Grundsätzlich ist es dabei auch denkbar, dass von mehreren Seiten Taschen in die Gepäckstange 10a eingebracht sind. Die vorangegangene Beschreibung dient lediglich zur genaueren Beschreibung eines möglichen Ausführungsbeispiels der Gepäckstange 10a, soll aber im Bezug auf die erfindungsgemäße Sitzvorrichtung nicht einschränkend gesehen werden.

Die Sitzvorrichtung weist ein Trägerelement 14a auf. Das Trägerelement 14a ist dazu vorgesehen, in zumindest einem Betriebszustand eine Abstützkraft aufzunehmen. Das Trägerelement 14a ist als eine gebogene flache Stange ausgebildet. Dabei weist das Trägerelement 14a einen Querschnitt auf, dessen Breite, die in montiertem Zustand parallel zu der Querrichtung des Sitzes 12a ausgerichtet ist, wesentlich größer ist als eine Höhe, die parallel zu einer Vertikalrichtung des Sitzes 12a verläuft. Das Trägerelement 14a ist zwischen dem Sitzteiler 26a und der Gepäckstange 10a angeordnet. Dabei weisen das Trägerelement 14a und die Gepäckstange 10a eine Koppelstelle 16a auf, die dazu vorgesehen ist, zumindest einen Teil der Abstützkraft von dem Trägerelement 14a auf die Gepäckstange 10a zu übertragen. Eine Kraft, insbesondere eine Abstützkraft, die während eines Betriebszustands in das Trägerelement 14a eingeleitet wird, wird über die Koppelstelle 16a in die Gepäckstange 10a und über deren Verbindung mit dem Grundrahmen des Sitzes 12a in den Sitz 12a und dadurch in den Kabinenboden, auf dem der Sitz 12a befestigt ist, eingeleitet. Das Trägerelement 14a ist dabei als ein Trittelement ausgebildet. Das als Trittelement ausgebildete Trägerelement 14a stellt einen von dem Kabinenboden erhöhten Trittbereich 50a bereit, auf dem beispielsweise ein Gast zum Erreichen von über dem Sitz 12a angeordneten Verstaumöglichkeiten auftreten kann, um einen erhöhten Stand zu haben. Grundsätzlich ist es aber auch denkbar, dass das Trägerelement 14a dazu vorgesehen ist, verschiedene Anbauteile, wie beispielsweise Lichtelemente, Halte- und/oder Sicherungselemente, aufzunehmen. Es ist beispielsweise denkbar, dass das Trägerelement 14a zur Befestigung von Lichtelementen vorgesehen ist, die einen Gang des Kabinenbodens beleuchten. Grundsätzlich ist es ebenfalls denkbar, dass das Trägerelement 14a zur Befestigung von mehreren, unterschiedlichen Elementen vorgesehen ist. Eine Abstützkraft, die das Trägerelement 14a dabei aufzunehmen hat, wird dabei von der Gewichtskraft der an dem Trägerelement 14a angebundenen Elemente gebildet. Grundsätzlich ist es auch denkbar, dass das Trägerelement 14a als Trittelement ausgebildet und dazu vorgesehen ist, weitere Elemente, wie beispielsweise Lichtelemente, an dem Sitz 12a zu befestigen.

Das Trägerelement 14a weist eine im Wesentlichen L-förmige Grundform auf. Von der Koppelstelle 16a aus, an der das Trägerelement 14a fest mit der Gepäckstange 10a gekoppelt ist, erstreckt sich das Trägerelement 14a im Wesentlichen parallel zu einem horizontal verlaufenden Bereich der Gepäckstange 10a in Richtung eines vorderen Bereichs des Sitzes 12a, also in Richtung der vorderen Querstange 32a. Der horizontal verlaufende Bereich des Trägerelements 14a, der von der Koppelstelle 16a mit der Gepäckstange 10a ausgeht, soll im Folgenden als Horizontalbereich 52a des Trägerelements 14a bezeichnet werden. An einem der Koppelstelle 16a abgewandten Ende des Horizontalbereichs 52a des Trägerelements 14a bildet das Trägerelement 14a eine Umlenkung von etwa 90 Grad aus, mittels der der Horizontalbereich 52a in einen Vertikalbereich 54a des Trägerelements 14a übergeht. Die Umlenkung, in der das Trägerelement 14a von dem Horizontalbereich 52a in den Vertikalbereich 54a übergeht, ist dabei von einem Radius gebildet. Der Vertikalbereich 54a des Trägerelements 14a erstreckt sich in montiertem Zustand von dem Kabinenboden weg in Richtung des entsprechenden Sitzteilers 26a und des Sitzbodens des entsprechenden Sitzes 12a. An einem der Koppelstelle 16a, an die das Trägerelement 14a mit der Gepäckstange 10a gekoppelt ist, abgewandten Ende bildet das Trägerelement 14a einen Anbindungsbereich 56a aus. Der Anbindungsbereich 56a ist zu dem Vertikalbereich 54a des Trägerelements 14a abgewinkelt und verläuft parallel zu einer Anbindungsstelle des Sitzteilers 26a, an die das Trägerelement 14a mittels des Anbindungsbereichs 56a angebunden ist. Der Anbindungsbereich 56a weist ein Durchgangsloch 58a auf, über das das Trägerelement 14a mittels einer Schraubenverbindung fest mit dem Sitzteiler 26a verbunden werden kann. In montiertem Zustand ist das Trägerelement 14a mittels eines Schraubenelements durch das Durchgangsloch 58a des Anbindungsbereichs 56a fest mit dem Sitzteiler 26a verbunden. Dazu weist der Sitzteiler 26a an der Anbindungsstelle ein entsprechend ausgebildetes Gewindeloch auf, in das das Schraubenelement zur Befestigung des Trägerelements 14a geschraubt wird. Grundsätzlich ist es auch denkbar, dass das Trägerelement 14a mittels eines anderen Befestigungselements, beispielsweise mittels eines anderen Formschlusselements, fest mit dem Sitzteiler 26a verbunden ist.

Das Trägerelement 14a ist über die Koppelstelle 16a formschlüssig mit der Gepäckstange 10a verbunden. Dazu umfasst das Trägerelement 14a ein Formschlusselement 18a, das die Gepäckstange 10a vollständig umschließt. An der der Gepäckstange 10a in montiertem Zustand zugewandten Seite des Trägerelements 14a ist das Formschlusselement 18a angeordnet. Das Formschlusselement 18a weist ein Durchgangsloch 60a auf, das korrespondierend zu dem Querschnitt der Gepäckstange 10a ausgebildet ist. Dabei weist das Durchgangsloch 60a eine Grundform auf, die etwas größer ist als der Querschnitt der Gepäckstange 10a, sodass die Gepäckstange 10a zur Montage des Trägerelements 14a durch das Durchgangsloch 60a des Formschlusselements 18a des Trägerelements 14a geführt werden kann. In montiertem Zustand ist die Gepäckstange 10a durch das Durchgangsloch 60a des Formschlusselements 18a des Trägerelements 14a geführt, wodurch das Formschlusselement 18a des Trägerelements 14a die Gepäckstange 10a komplett umschließt.

Die Gepäckstange 10a umfasst ein Formschlusselement 20a, das zur Anbindung des Trägerelements 14a an die Gepäckstange 10a vorgesehen ist. Das Formschlusselement 20a ist dabei korrespondierend zu dem Formschlusselement 18a des Trägerelements 14a ausgebildet. Das Formschlusselement 18a des Trägerelements 14a und das Formschlusselement 20a der Gepäckstange 10a, über die das Trägerelement 14a fest mit der Gepäckstange 10a verbunden ist, bilden zusammen die Koppelstelle 16a aus. Das Formschlusselement 20a bildet zwei an die Gepäckstange 10a angeformte Erhebungen 22a, 24a aus. Die an die Gepäckstange 10a angeformten Erhebungen 22a, 24a sind dabei an breiten Seiten der als flache Stange ausgebildeten Gepäckstange 10a angeordnet, die orthogonal zu der Querrichtung des Sitzes 12a ausgerichtet sind. Die Erhebungen 22a, 24a des Formschlusselements 20a der Gepäckstange 10a vergrößern im Bereich des Formschlusselements 20a den Querschnitt der Gepäckstange 10a, der dort dann größer ist als das Durchgangsloch 60a des Formschlusselements 18a des Trägerelements 14a. Dadurch liegt das Formschlusselement 18a des Trägerelements 14a in montiertem Zustand auf den Erhebungen 22a, 24a des Formschlusselements 20a der Gepäckstange 10a auf. Das Trägerelement 14a liegt mit einer Unterseite, die in montiertem Zustand dem Kabinenboden zugewandt ist, auf Abstützflächen der Erhebungen 22a, 24a des Formschlusselements 20a der Gepäckstange 10a auf, die dem Kabinenboden abgewandt sind. Durch das Aufliegen des Trägerelements 14a auf den Erhebungen 22a, 24a des Formschlusselements 20a der Gepäckstange 10a ist das Trägerelement 14a in Richtung des Kabinenbodens formschlüssig mit der Gepäckstange 10a verbunden. Grundsätzlich ist es auch denkbar, dass das Formschlusselement 20a eine um die Gepäckstange 10a umlaufende Erhebung aufweist.

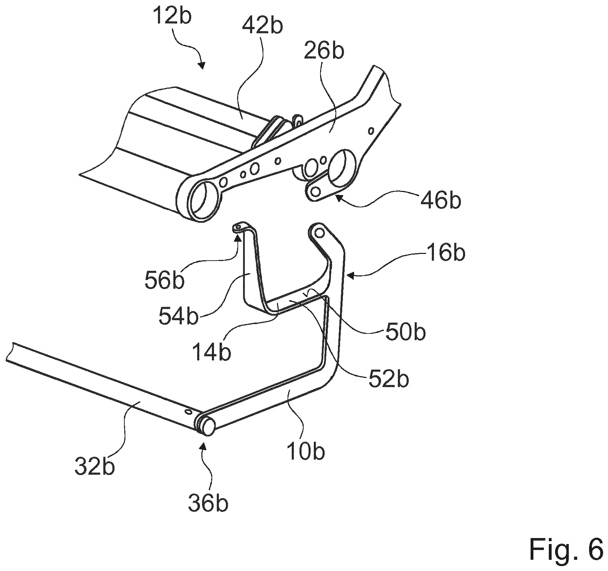

In Fig. 6 ist ein weiteres Ausführungsbeispiel der Erfindung gezeigt. Die nachfolgenden Beschreibungen und die Zeichnungen beschränken sich im Wesentlichen auf die Unterschiede zwischen den Ausführungsbeispielen, wobei bezüglich gleich bezeichneter Bauteile, insbesondere in Bezug auf Bauteile mit gleichen Bezugszeichen, grundsätzlich auch auf die Zeichnungen und/oder die Beschreibung der anderen Ausführungsbeispiele, insbesondere der Fig. 1 bis 5, verwiesen werden kann. Zur Unterscheidung der Ausführungsbeispiele ist der Buchstabe a den Bezugszeichen des Ausführungsbeispiels in Fig. 1 bis 5 nachgestellt. In dem Ausführungsbeispiel der Fig. 6 ist der Buchstabe a durch den Buchstaben b ersetzt.

Die Figur 6 zeigt ein zweites Ausführungsbeispiel einer erfindungsgemäßen Sitzvorrichtung. Die Sitzvorrichtung umfasst eine seitliche Gepäckstange 10b. Die seitliche Gepäckstange 10b ist Teil des als Flugzeugsitz ausgebildeten Sitzes 12b, der mit einer Seite an einen Gang in der Passagierkabine grenzt. Die Sitzvorrichtung weist ein Trägerelement 14b auf. Das Trägerelement 14b ist dazu vorgesehen, in zumindest einem Betriebszustand eine Abstützkraft aufzunehmen. Das Trägerelement 14b ist zwischen einem Sitzteiler 26b des Sitzes 12b und der Gepäckstange 10b angeordnet. Dabei weisen das Trägerelement 14b und die Gepäckstange 10b eine Koppelstelle 16b auf, die dazu vorgesehen ist, zumindest einen Teil der Abstützkraft von dem Trägerelement 14b auf die Gepäckstange 10b zu übertragen. Das Trägerelement 14b ist dabei als ein Trittelement ausgebildet. Im Unterschied zu der Ausgestaltung aus dem ersten Ausführungsbeispiel der Figuren 1 bis 5 sind das Trägerelement 14b und die Gepäckstange 10b einstückig miteinander ausgebildet. Das Trägerelement 14b und die Gepäckstange 10b sind aus einem gemeinsamen, zusammenhängenden Bauteil gebildet. Dabei sind das Trägerelement 14b und die Gepäckstange 10b vorzugsweise einstückig aus einem Rohling geformt, beispielsweise in einem Gießverfahren. Grundsätzlich ist es auch denkbar, dass die Gepäckstange 10b und das Trägerelement 14b mittels eines Schweißverfahrens oder mittels eines anderen, dem Fachmann als sinnvoll erscheinenden Verfahrens, stoffschlüssig miteinander verbunden sind. Grundsätzlich ist es auch denkbar, dass das Trägerelement 14b lediglich mit der Gepäckstange 10b verbunden ist. Dabei wäre das Trägerelement 14b nicht mit dem Sitzteiler 26b verbunden und eine gesamte in das Trägerelement 14b eingeleitete Abstützkraft würde über die Koppelstelle 16b in die Gepäckstange 10b eingeleitet.

### Bezugszeichen

- 10: Gepäckstange
- 12: Sitz
- 14: Trägerelement
- 16: Koppelstelle
- 18: Formschlusselement
- 20: Formschlusselement
- 22: Erhebung
- 24: Erhebung
- 26: Sitzteiler
- 28: Aufständereinheit
- 30: Abdeckung
- 32: Querstange
- 34: vorderes Ende
- 36: Verbindungsstelle
- 38: Formschlusselement
- 40: Formschlusselement
- 42: Tragrohr
- 44: Umlenkung
- 46: hinteres Ende
- 48: Schraubenverbindung
- 50: Trittbereich
- 52: Horizontalbereich
- 54: Vertikalbereich
- 56: Anbindungsbereich
- 58: Durchgangsloch
- 60: Durchgangsloch

## Patentansprüche

1. Sitzvorrichtung, insbesondere Flugzeugsitzvorrichtung, mit zumindest einer seitlichen Gepäckstange (10a; 10b), die dazu vorgesehen ist, unterhalb eines Sitzes (12a; 12b) an einer, einem Gang einer Passagierkabine zugewandten Seite des Sitzes (12a; 12b) angebracht zu werden, wobei sich die seitliche Gepäckstange (10a; 10b) von ihrem vorderen Ende (34a; 34b) aus im Wesentlichen parallel zu dem Kabinenboden in Richtung eines hinteren Bereichs des Sitzes (12a; 12b) erstreckt, und mit zumindest einem Trägerelement (14a; 14b), das dazu vorgesehen ist, eine Abstützkraft aufzunehmen, **dadurch gekennzeichnet, dass** das Trägerelement (14a; 14b) von einem Trittelement gebildet ist, das einen von einem Kabinenboden erhöhten Trittbereich (50a) bereitstellt, und dass das Trägerelement (14a; 14b) und die Gepäckstange (10a; 10b) zumindest eine Koppelstelle (16a; 16b) aufweisen, über die die Gepäckstange (10a; 10b) und das Trägerelement (14a; 14b) form-, stoff- und/oder kraftschlüssig miteinander verbunden sind und die dazu vorgesehen ist, zumindest einen Teil der Abstützkraft von dem Trägerelement (14a; 14b) auf die Gepäckstange (10a; 10b) zu übertragen.

2. Sitzvorrichtung nach Anspruch 1, **dadurch gekennzeichnet, dass** das Trägerelement (14a; 14b) eine im Wesentlichen L-förmige Grundform aufweist.

3. Sitzvorrichtung nach einem der vorhergehenden Ansprüche, **dadurch gekennzeichnet, dass** das Trägerelement (14a) ein Formschlusselement (18a) umfasst, das die Gepäckstange (10a) zur formschlüssigen Verbindung zumindest im Wesentlichen umschließt.

4. Sitzvorrichtung nach Anspruch 3, **dadurch gekennzeichnet, dass** die Gepäckstange (10a) zumindest ein Formschlusselement (20a) umfasst, das korrespondierend zu dem Formschlusselement (18a) des Trägerelements (14a) ausgebildet ist.

5. Sitzvorrichtung nach Anspruch 4, **dadurch gekennzeichnet, dass** das zumindest eine Formschlusselement (20a) der Gepäckstange (10a) zumindest eine an die Gepäckstange (10a) angeformte Erhebung (22a, 24a) aufweist.

6. Sitzvorrichtung nach einem der vorhergehenden Ansprüche, **dadurch gekennzeichnet, dass** die Gepäckstange (10a; 10b) zumindest teilweise von einem Vollprofil gebildet ist.

7. Sitzvorrichtung nach einem der vorhergehenden Ansprüche, **dadurch gekennzeichnet, dass** die Gepäckstange (10a; 10b) zumindest teilweise als eine flache Stange ausgebildet ist.

8. Sitzvorrichtung nach einem der vorhergehenden Ansprüche, **gekennzeichnet durch** einen Sitzteiler (26a; 26b), wobei das Trägerelement (14a; 14b) zwischen dem Sitzteiler (26a; 26b) und der Gepäckstange (10a; 10b) angeordnet ist.

9. Sitzvorrichtung nach einem der vorhergehenden Ansprüche, **dadurch gekennzeichnet, dass** die Gepäckstange (10b) und das Trägerelement (14b) einstückig miteinander ausgebildet sind.

10. Sitz, insbesondere Flugzeugsitz, mit einer Sitzvorrichtung nach einem der vorhergehenden Ansprüche.

## Claims

1. Seating device, in particular airplane seating device,
with at least one lateral baggage bar (10a; 10b), which is configured to be mounted underneath a seat (12a; 12b) on a side of the seat (12a; 12b) facing toward an aisle of a passenger cabin, the lateral baggage bar (10a; 10b) extending from its front end (34a; 34b) at least substantially parallel to the cabin floor toward a rear region of the seat (12a; 12b),
and with at least one carrier element (14a; 14b) that is configured to receive a support force,
**characterised in that** the carrier element (14a; 14b) is realized by a step element providing a stepping zone (50a) that is elevated with respect to the cabin floor,
and that the carrier element (14a; 14b) and the baggage bar (10a; 10b) have at least one coupling point (16a; 16b) via which the baggage bar (10a; 10b) and the carrier element (14a; 14b) are connected to each other in a form-fit and/or force-fit manner and/or by substance-to-substance bond, the coupling point (16a; 16b) being configured to transfer at least a portion of the support force from the carrier element (14a; 14b) to the baggage bar (10a; 10b).

2. Seating device according to claim 1,
**characterised in that** the carrier element (14a; 14b) has an at least substantially L-shaped basic shape.

3. Seating device according to one of the preceding claims,
**characterised in that** the carrier element (14a) comprises a form-fit element (18a) which at least substantially encompasses the baggage bar (10a) for the purpose of a form-fit connection.

4. Seating device according to claim 3,
**characterised in that** the baggage bar (10a) comprises at least one form-fit element (20a), which is implemented correspondingly to the form-fit element (18a) of the carrier element (14a).

5. Seating device according to claim 4,
**characterised in that** the at least one form-fit element (20a) of the baggage bar (10a) has at least one bump (22a, 24a) that is moulded to the baggage bar (10a).

6. Seating device according to one of the preceding claims,
**characterised in that** the baggage bar (10a; 10b) is formed at least partly by a solid profile.

7. Seating device according to one of the preceding claims,
**characterised in that** the baggage bar (10a; 10b) is embodied at least partly as a flat bar.

8. Seating device according to one of the preceding claims,
**characterised by** a seat divider (26a; 26b), the carrier element (14a; 14b) being arranged at least partly between the seat divider (26a; 26b) and the baggage bar (10a; 10b).

9. Seating device according to one of the preceding claims,
**characterised in that** the baggage bar (10b) and the carrier element (14b) are implemented integrally with each other.

10. Seat, in particular airplane seat, with a seating device according to one of the preceding claims.

## Revendications

1. Dispositif à siège, notamment dispositif à siège d'aéronef,
avec au moins une barre à bagages (10a ; 10b) latérale, qui est prévue à être montée au-dessous d'un siège (12a ; 12b) sur un côté du siège (12a ; 12b) tourné vers une allée d'une cabine des passagers,
la barre à bagages (10a ; 10b) latérale s'étendant à partir de son extrémité avant (34a ; 34b) au moins sensiblement en parallèle au plancher de cabine vers une zone arrière du siège (12a ; 12b),
et avec au moins un élément porteur (14a ; 14b), prévu à recevoir une force de support,
**caractérisé en ce que** l'élément porteur (14a ; 14b) est formé par un élément marchepied pourvoyant une zone marchepied (50a) élevée par rapport au plancher de cabine,
et que l'élément porteur (14a ; 14b) et la barre à bagages (10a ; 10b) comprennent au moins un point de couplage (16a ; 16b) par le biais duquel la barre à bagages (10a ; 10b) et l'élément porteur (14a ; 14b) sont raccordés l'une à l'autre par liaison en forme, en matière et/ou en force, et qui est prévu à transférer au moins une partie de la force de support de l'élément porteur (14a ; 14b) à la barre à bagages (10a ; 10b).

2. Dispositif à siège selon la revendication 1,
**caractérisé en ce que** l'élément porteur (14a ; 14b) présente une forme basique sensiblement en forme de L.

3. Dispositif à siège selon l'une des revendications précédentes,
**caractérisé en ce que** l'élément porteur (14a) comporte un élément de liaison en forme (18a) au moins sensiblement entourant la barre à bagages (10a) pour la liaison en forme.

4. Dispositif à siège selon la revendication 3,
**caractérisé en ce que** la barre à bagages (10a) comporte au moins un élément de liaison en forme (20a) réalisé en correspondance à l'élément de liaison en forme (18a) de l'élément porteur (14a).

5. Dispositif à siège selon la revendication 4,
**caractérisé en ce que** l'au moins un élément de liaison en forme (20a) de la barre à bagages (10a) comporte au moins une élévation (22a, 24a) moulée sur la barre à bagages (10a).

6. Dispositif à siège selon l'une des revendications précédentes,
**caractérisé en ce que** la barre à bagages (10a ; 10b) est réalisée au moins partiellement par un profilé massif.

7. Dispositif à siège selon l'une des revendications précédentes,
**caractérisé en ce que** la barre à bagages (10a ; 10b) est formée au moins partiellement comme barre plate.

8. Dispositif à siège selon l'une des revendications précédentes,
**caractérisé par** un séparateur de sièges (26a ; 26b), l'élément porteur (14a ; 14b) étant disposé entre le séparateur de sièges (26a ; 26b) et la barre à bagages (10a ; 10b).

9. Dispositif à siège selon l'une des revendications précédentes,
**caractérisé en ce que** la barre à bagages (10b) et l'élément porteur (14b) sont réalisés intégralement l'une avec l'autre.

10. Siège, en particulier siège d'aéronef, avec un dispositif de siège selon l'une des revendications précédentes.
